# EUROPÄISCHE PATENTSCHRIFT

(11) **EP 0 446 308 B1**
(45) Veröffentlichungstag und Bekanntmachung des Hinweises auf die Patenterteilung: **14.09.1994**
(21) Anmeldenummer: 90904790.4
(22) Anmeldetag: 02.04.1990
(51) Int. Cl.: A61F 5/01

(54) **Knieorthese und Verfahren zu deren Herstellung**
Knee Orthesis and process for its manufacture
Orthese de genou et procédé pour fabrication

(30) Priorität: 30.09.1989 CH 3544/89
(43) Veröffentlichungstag der Anmeldung: 18.09.1991
(73) Patentinhaber: TAMAGNI AG, CH-8008 Zürich (CH)
(72) Erfinder: BOESCH, René, CH-8962 Viganello (CH)
(74) Vertreter: Schwabe, Hans-Georg, Dipl.-Ing.
(86) Internationale Anmeldenummer: CH9000085
(87) Internationale Veröffentlichungsnummer: WO9104720

(56) Entgegenhaltungen:
- AT-B- 369 978
- AT-B- 384 733
- FR-A- 1 236 669
- GB-A- 1 348 781
- Medizinisch-Orthopädische Technik, vol. 108,No.6, November/December 1988, Gentner publisher (Stuttgart, DE), J. Eichler: "Knieorthesen", pages 201-212, page 206,paragraph 8. "vierfach-Gelenke"; pages 208-209: "C.T.I.-knieorthese", figures 13, 16

## Beschreibung

Die Erfindung betrifft eine Knieorthese bzw. ein Verfahren zur Herstellen eines orthopädischen Stützapparates in der Form einer Knieorthese gemäß dem Oberbegriff des Patentanspruchs 1 bzw. der Patentansprüche 11 bzw. 12.

Zur Stabilisierung des Bandapparates im Knie werden Knieorthesen verwendet.

Während früher Knieorthesen vor allem präoperativ als Verhütungsmassnahme gegen weitere Schäden bei angerissenen oder überspannten Bändern sowie postoperativ nach Bänderoperationen zur Schonung verschrieben wurden, verwenden heute Sportler aus den verschiedensten Disziplinen diese Knieorthesen mehr oder weniger freiwillig, um vorbeugend Knie- oder Bänderverletzungen zu verhindern.

Die einfachste Ausführung der Knieorthese besitzt ein monozentrisches Gelenk; mit diesem lässt sich aber die anatomische Bewegung des Kniegelenkes nicht richtig nachbilden. Die Bewegung beziehungsweise das Zusammenspiel aller an der anatomischen Bewegung beteiligten Elemente des Knies (Gelenkflächen des Femurs und der Tibia, Kreuzbänder, Patella) ist viel komplexer. Das führt dazu, dass Knieorthesen mit monozentrischen Gelenken sich nicht gleichzeitig synchron mit dem Oberschenkel und dem Schienbein mitbewegen können. Die Folge sind lästige Reibungen, welche den Tragkomfort mindern. Da monozentrische Gelenke aus anatomischen Gesichtspunkten sowieso eine Behelfslösung darstellen, sind sie gerade für Sportler ungeeignet.

Die tatsächlich physiologisch auftretende Roll-Gleit-Bewegung der Kniegelenkflächen und die besondere Lage der Kreuzbänder wurde durch Menschik studiert und beschrieben. Das Ergebnis ist eine nach ihm benannte sogenannte "überschlagene Viergelenkkette" (im folgenden einfach Viergelenkkette genannt), welche die tatsächliche Roll-Gleit-Bewegung sehr gut nachbildet und somit das Knie wirksam entlastet. Damit ist aber noch lange nicht ein Knieorthese gebaut, die einen hohen Tragkomfort und gleichzeitig eine gute Führung des Bandapparats vereinigt; Voraussetzungen, die im heutigen Hochleistungssport als selbstverständlich angesehen werden.

Gegenwärtig existieren auf dem Markt Fertigbauschienen für den Anbau an separat erhältliche Viergelenkketten. Diese Knieorthesen werden mit Konfektionsbandagen am Kniegelenk befestigt. Die Konfektionsbandagen weisen Taschen auf, in welche die Fertigbauschienen gesteckt werden. Die Schienen haben allerdings in den Taschen ein nicht zu vernachlässigendes Spiel, bewegen sich also hin und her, weshalb mit dieser Knieorthese das Knie schlecht geführt ist.

Eine ähnliche Knieorthese wird in der Druckschrift "Medizinisch-orthopädische Technik", Band 108, Nr. 6, 1988, auf Seite 206 und Seite 208 beschrieben. Diese Knieorthese besteht aus einer Kniebandage mit einem seitlich in einer Tasche befindlichen Viergelenkgetriebe. Die erwünschte Sicherheit ist auch in diesem Fall für den Bandapparat bei starken oder gar extremen Belastungen nicht gegeben.

Derselben Druckschrift ist auf Seite 206 eine Knieorthese mit dreiachsigen Gelenken zu entnehmen. Durch dieses Gelenk werden eine obere und eine untere Kunststoffhalbschale verbunden, die jeweils in vier Größen geliefert werden. Das Gelenk ist außen an der Kunststoffhalbschale angebracht. Unvorteilhafterweise weist diese Knieorthese ein dreiachsiges Gelenk und nicht ein vierachsiges Gelenk auf. Zudem sind die Kunststoffschalen, da sie nur in vier Größen geliefert werden, nicht paßgenau und damit nicht rutschfest. Eine individuell angepaßte Positionierung des dreiachsigen Gelenks ist deshalb nur bedingt möglich.

Um nun eine exaktere Paßform zu erreichen, werden die modernsten Fertigungstechniken voll ausgeschöpft: Mit CAD/CAM-Unterstützung können Knieschienen aus einem massiven Karbonfasermonoblock herausgefräst werden, nachdem die Abmessungen des Kniegelenks und der angrenzenden Partien des Ober- und Unterschenkels mit einem Laserscanner erfaßt und digitalisiert worden sind. Allerdings sind die auf diese Weise hergestellten Knieorthesen ohne Viergelenkketten ausgerüstet und haben den entscheidenden Nachteil, daß sie wegen des enormen technischen Aufwandes (integrierte CAD/CAM-Fertiginsel) sehr teuer sind.

Aus der Druckschrift FR-A-1 236 669 ist das Eingießen eines Arms eines Scharniergelenks in Kunststoffschichten zu orthopäischen Zwecken bekannt. Durch diese Scharniergelenke kann jedoch nicht der natürliche Bewegungsablauf nachgebildet werden.

In der Druckschrift GB-A-1 348 781 wird ein Verfahren zur Herstellung eines orthopädischen Stützapparates, zum Stützen von beispielsweise menschlichen Gliedmaßen, beschrieben, bei dem zunächst ein positiver Gipsabdruck hergestellt, dann eine Karbonverbundschicht auf den Gipsabdruck aufgebracht, mit einem Laminat ausgegossen und hernach ausgehärtet wird und schließlich nach Entfernen des Gipsabdrucks die endgültige Form des Stützapparats zugeschnitten wird.

Es ist die Aufgabe der vorliegenden Erfindung, eine Knieorthese zur Verfügung zu stellen bzw. herzustellen, die einen hohen Tragekomfort und gleichzeitig eine gute Führung des Bandapparates vereinigt.

Diese Aufgabe wird durch die Gegenstände mit den in den Patentansprüchen 1, 11 und 12 aufgeführten Merkmalen gelöst.

Zweckmäßige Ausgestaltungen bzw. Verfahrensvarianten gehen aus den Unteransprüchen hervor.

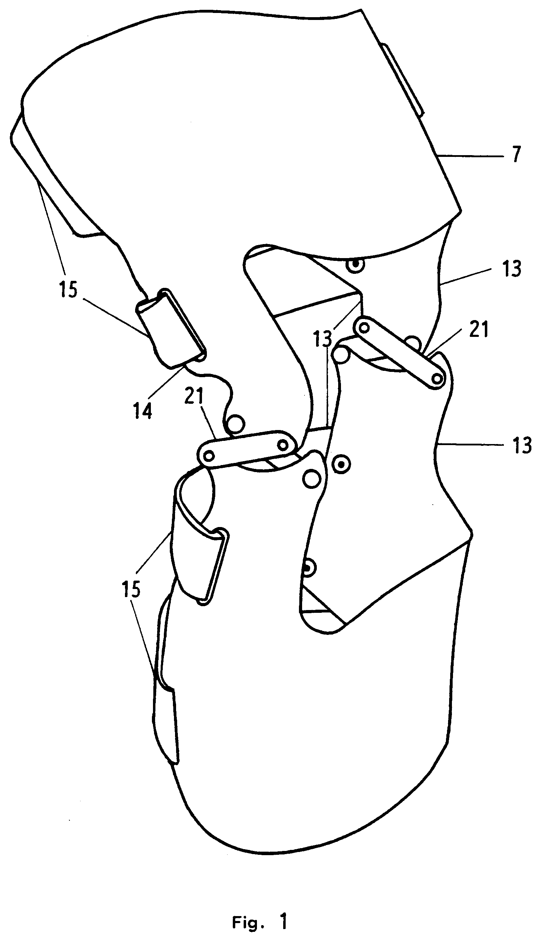

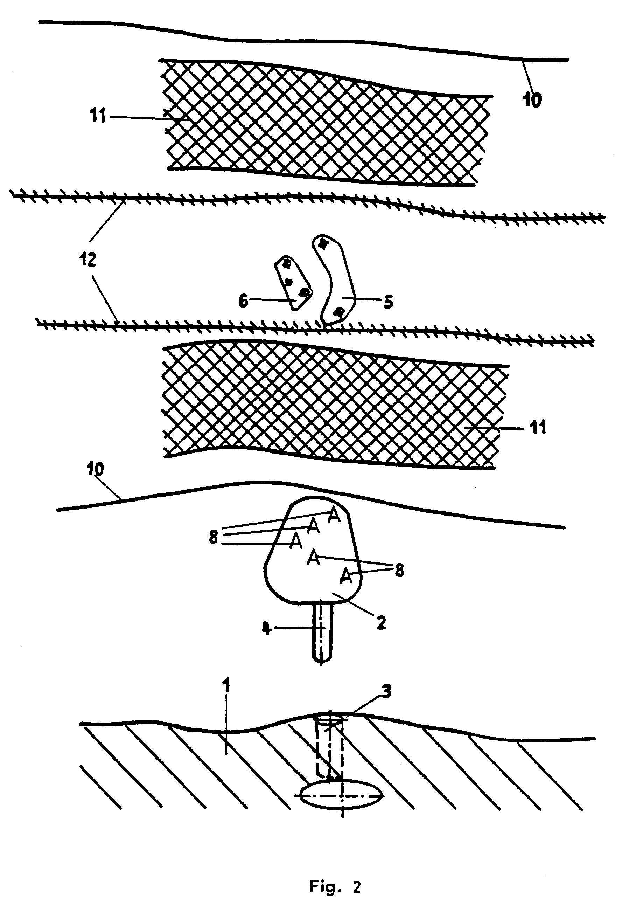

Die neuen Knieorthesen in Karbon-Verbundschichtbauweise sind äusserst passgenau und erfüllen ihren Zweck dank integrierten Viergelenkketten nach Menschik auf hervorragende Art und Weise. Es Zeigen:
- Fig. 1: Gesamtamsicht der Knieorthese
- Fig. 2: Aufbau/Herstellung der Knieorthese
- Fig. 3: Teile der Viergelenkkette
- Fig. 4: Viergelenkkette montiert, Wirkungsweise
Herausragende zusätzliche Merkmale der neuen Knieorthese 7 (Fig. 1) in Karbon-Verbundschichtbauweise sind ihr geringes Gewicht (ca. 360 g) und die sehr einfach mögliche Herstellung ab einem einfachen positiven Gipsabdruck 1 (Fig. 2) des Knies. Dadurch, dass ein einfacher Gipsabdruck 1 als Grundlage dient, ist gewährleistet, dass die Karbon-Verbundorthese 7 in dazu ausgerüsteten orthopädischen Werkstätten zu tiefen Preisen und in gleichbleibend guter Qualität hergestellt werden kann.

Im Detail geht das wie folgt vor sich: Es wird als erstes ein negativer Gipsabdruck des Beines hergestellt, das die Knieorthese 7 tragen wird. Die Knieorthese wird drei Viertel des Beines einfassen. Der negative Gipsabdruck wird nach dem Aushärten lateral aufgeschnitten und vom Bein entfernt. Anschliessend wird er wieder zusammengefügt und mit Gipsbinde verschlossen. Dieses Negativ wird nun seinerseits wieder mit flüssigem Gips ausgegossen, wodurch man einen positven Gipsabdruck 1 (Fig. 2) und somit eine exakte Nachbildung des Kniegelenks des Patienten erhält. Durch anschliessende Modellierung können etwaige Unregelmässigkeiten ausgeglichen werden.

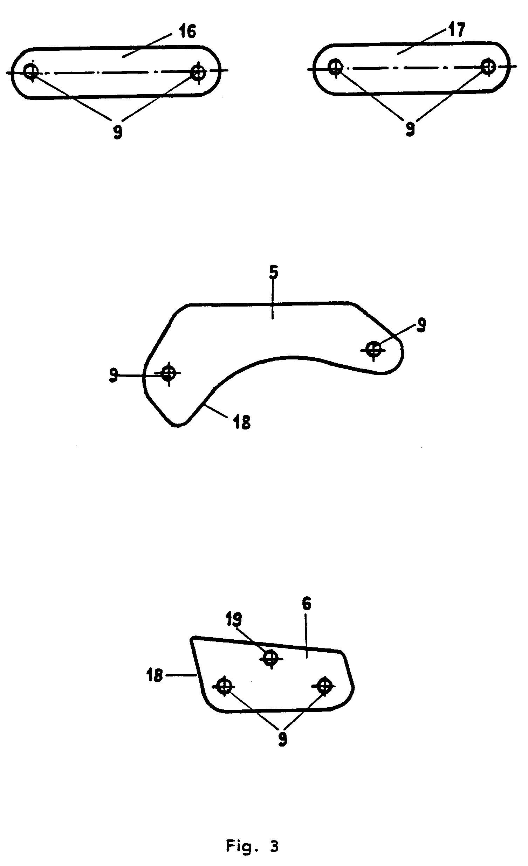

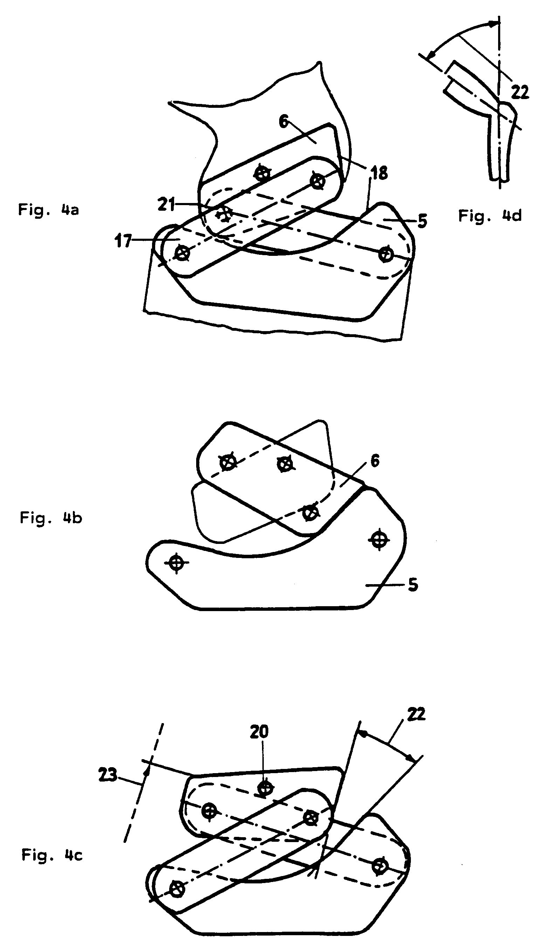

Jetzt werden im positiven Gipsabdruck 1 im Lateral- und Medialbereich des Kniegelenkes, in welchem sich die Viergelenkketten 21 (Fig. 4a) befinden müssen, zwei Grundrichtplatten 2 (Fig. 2) eingesetzt. Zu diesem Zweck werden in den positiven Gipsabdruck 1 zwei Löcher 3 gebohrt, in welche die Haltestifte 4 der Grundrichtplatten 2 eingesteckt werden (Fig. 2). Entscheidend ist nun, dass in einem (später beschriebenen) Arbeitsgang die sichelförmige untere Gelenkplatte 5 (Fig. 3) und die obere trapezoidförmige Gelenkplatte 6 der Viergelenkkette dank der Grundrichtplatte 2 vorpositioniert und in die eigentliche Karbonverbund-Knieorthese 7 (Fig. 1) voll integriert werden können. Die schliesslich resultierenden integrierten Viergelenkketten 21 sind absolut spielfrei und exakt positioniert, was eine bisher noch nicht gekannte Sicherheit für den Bandapparat des Kniegelenks gewährleistet. Dabei versteht es sich von selbst, dass auch der Tragkomfort deutlich erhöht wird; eine hohe Passgenauigkeit lässt den Patienten die Orthese vergessen. Es sei an dieser Stelle noch darauf hingewiesen, dass die Grundrichtplatten 2 zur Positionierung der Gelenkplatten 5,6 mit Positionierstiften 8 (Fig. 2) versehen sind, deren Lage mit den Bohrungen 9 (Fig. 3) der Gelenkplatten 5,6 übereinstimmen. Auf die zusätzliche Bedeutung der Grundrichtplatten wird später eingegangen.

Im weiteren wird der Hohlraum, der sich zwischen dem Gipsabdruck 1 und den eingesteckten Grundrichtplatten 2 zwangsläufig bildet, mit Plastilin ausgefüllt. Bevor nun mit der Herstellung der Karbon-Verbundschicht begonnen wird, muss der positive Gipsabdruck 1 mit einem (in Fig. 2 nicht gezeichneten) Baumwolltrikot überzogen werden. Dieses Baumwolltrikot wird am Schluss entfernt und hat lediglich die Aufgabe, die Restfeuchtigkeit des Gipsabdruckes 1 von der als erste Lage über den Gipsabdruck angelegte Polyvinylacetat-Folie 10 (PVA-Folie) fernzuhalten. Jetzt folgt eine Karbonlage 11. Dabei handelt es sich um eine Kohlefasergeflechtmatte mit zweihundert Flechtungen pro Quadratzentimeter. Diese Matte wird axial um den Gipsabdruck 1 gelegt und auf der Rückseite (also "hinten" am Bein) zusammengeklebt. Nun kann das zur mechanischen Verstärkung dienende schlauchförmige Glasfasertrikot 12 übergezogen werden. Die erste Hälfte der Karbonverbundschicht ist nun vorbereitet. Die Gelenkplatten 5,6 (Fig. 2) können nun wie oben erwähnt auf die Positionierstifte 8 der Grundrichtplatte 1 aufgesteckt werden. Das ist sehr einfach möglich, weil die Positionierstifte die dünne PVA-Folie 10 sowie die Karbonlage 11 und das Glasfasertrikot 12 gut durchdringen. Wichtig ist nun, dass die Gelenkplatten 5,6 auf dem darunterliegenden Glasfasertrikot aufgeklebt werden, damit sie sich nachträglich nicht verschieben können. Glasfasertrikot hat übrigens im Vergleich zu Glasfasermatte den Vorteil, dass es sich beim Ziehen nicht verfasert. Das wirkt sich direkt als Qualitätssteigerung des Karbonverbunds aus, da die tragende Matte, mechanisch gesehen, keine Unterbrüche aufweist, welche die Tragfähigkeit der Karbonverbundschicht beeinträchtigen würden.

Jetzt wird die zweite, äussere Hälfte der Karbonverbundschicht geschichtet, indem eine zweite Lage Glasfasertrikot 12 (Fig. 2) übergezogen wird. Um den Bereich der Viergelenkketten mechanisch zu versteifen (Erzielung der gewünschten Stabilität der Orthesengelenkzonen), wird der Gelenkbereich mit (in Fig. 2 nicht gezeichneten) Glasfassermatten zweimal übers Kreuz umwickelt. Anschliessend folgt ein weiteres, über alles reichendes Glasfasertrikot 12. Ueber dieses wird wiederum eine Lage Karbongeflecht 11 gelegt, auf welche die Deckschicht aus PVA-Folie 10 gelegt wird. Zusammenfassend hat man also einen "Sandwichverbund" erhalten, dessen innere Gewebeschichten zwischen zwei PVA-Folien um den Gipsabdruck 1 gelegt sind.

Zwischen diese zwei Folien wird nun flüssiges Laminat (Harz und Härter) gegossen. Die ausgegossene Rohform wird anschliessend an ein Vakuumgerät angeschlossen und die Luft evakuiert. Dabei wird zum einen das Schichtpaket fest auf den Gipsabdruck 1 gepresst und zum anderen die Luft zwischen den einzelnen Gewebeschichten vollständig entfernt; die Gewebeschichten werden durch das nachfliessende Laminat gleichmässig durchtränkt. Dieses Verfahren ist als Vakuumverfahren bestens bekannt und eignet sich erfahrungsgemäss ausgezeichnet für Einzelanfertigungen auf Gipsformen. Nun erklärt sich von selbst, weshalb wie oben erwähnt, der Hohlraum zwischen den Grundrichtplatten 2 und dem positiven Gipsabdruck 1 mit Plastilin ausgefüllt wird; es muss verhindert werden, dass durch das Evakuieren der sonst vorhandenen Luftblase die innere PVA-Folie reisst, was durch nachfliessendes Laminat dazu führen würde, dass die Grundrichtplatten 2 mit eingegossen würden. Das Aushärten erfolgt beim Vakuumverfahren bei Raumtemperatur und dauert etwa eine halbe Stunde. Die Orthesenschale ist nun bereit für die Endbearbeitung.

Als erstes werden die Kniegelenkkonturen 13 (Fig. 1) auf der Vorder- und Rückseite der Knieorthese ausgeschnitten. Damit werden die Teile des Knies freigelegt, die sich bewegen müssen. Die Knieorthese kann jetzt auch auf die endgültige Länge gekürzt werden. Der Gipsabdruck 1 ist danach nicht mehr nötig und wird später, nach Lieferung der Orthese, zerstört. Die (wiederverwendbaren) Grundrichtplatten werden herausgenommen. Das Baumwolltrikot und das Plastilin werden ebenfalls entfernt. Als Ergebnis erhält man die Knieorthese als noch starres Halbfabrikat. Die Orthese wird zwischen den beiden Gelenkplatten 5,6 durchgesägt, sodass man die obere und untere Hälfte für sich bearbeiten kann. Zum Beispiel kann nun überschüssiger Karbonverbundwerkstoff abgeschliffen werden, vor allem zwischen den Gelenkplatten. Auf der Rückseite werden die beiden Hälften längs geschlitzt, damit man die Knieorthese auch anziehen kann. Durchführungsschlitze 14 für die Klettverschlüsse 15 werden ausgestanzt und zur Erhöhung des Tragkomforts wird ein (nicht gezeigtes) Schienbeinpolster eingelegt. Abschliessend werden noch die inneren Schenkel 16 und die äusseren Schenkel 17 (Fig. 3) mit den integrierten Gelenkplatten zu den fertigen Viergelenkketten 21 (Fig. 4) und gleichzeitig zur fertigen Knieorthese 7 (Fig. 1) vernietet.

Zum geringen Traggewicht von ca. 360 g trägt die Tatsache bei, dass die Schenkel 16,17 und die Gelenkplatten 5,6 aus Titan bestehen, das bekanntlich beste Festigkeitswerte bei erstaunlich geringem Gewicht vereinigt. Die Verwendung von Karbonverbundwerkstoff für die Knieorthese ergibt ebenfalls geringstes Gewicht bei sehr hoher Festigkeit im Verhältnis zur notwendigen Materialdicke.

Um die weiteren Vorteile der neuen Knieorthese zu erkennen, müssen die Grundrichtplatte 2 und die Funktion der Viergelenkkette 21 (Fig. 4a) näher betrachtet werden. Wie erwähnt, werden dank der Grundrichtplatte 2 (Fig. 2) die Gelenkplatten 5,6 positioniert. Die gegenseitige Lage der Gelenkplatten bei der Herstellung der Knieorthese 7 hängt davon ab, wie stark das Knie des Patienten gestreckt werden kann. Ideal wäre ein gestrecktes Bein mit einem entsprechenden Beugewinkel von 0°, was aber nach einer Operation nie erreicht wird. (Der Beugewinkel 22 (Fig. 4d) ist der gedachte Winkel zwischen der Achse des Oberschenkels und der gedachten Verlängerung über das Knie hinaus des Unterschenkels.) Aus diesem Grund werden Grundrichtplatten mindestens für die Beugewinkel 20°, 30°,40° und 45° hergestellt. Selbstverständlich sind auch Grundrichtplatten für jeden beliebigen Beugewinkel 22 möglich. Damit ist gewährleistet, dass die Gelenkplatten 5,6 beim Fertigen der Knieorthese so liegen, wie es dem gegenwärtigen Beugewinkel des Patienten entspricht, bei welchem der Gipsabdruck 1 hergestellt wurde.

Die neue Knieorthese kann ausserdem nicht überstreckt werden (negativer Beugewinkel). Figur 4b zeigt, dass bei ganz gestrecktem Bein (Beugewinkel 0°) die gerade Flanke 18 der sichelförmigen unteren Gelenkplatte 5 (Fig. 3) als Anschlag für die Flanke 18 der oberen Gelenkplatte 6 dient. Ferner müssen die jeweils gegenüberliegenden Gelenkplatten der einzelnen Viergelenkketten exakt parallel und deckungsgleich ausgerichtet sein, um beim Biegen des Knies ein sogenanntes Aufklappen der Knieorthese zu verhindern. Trotz sorgfältigster Fertigung wird es aber Lagetoleranzen geben; diese können sehr einfach durch Nachschleifen der Anschlagflanken 18 (Fig. 3) ausgeglichen werden. Ein weiterer entscheidender Vorteil der neuen Knieorthese ist der, dass auf einfache Art und Weise der Beugewinkel 22 begrenzt werden kann. Das ist jeweils dann notwendig, wenn der Chirurg zum Beispiel verschreibt, der Patient dürfe sein Knie lediglich in einem Winkel zwischen 20° und 60° biegen. Die erste Möglichkeit, den Winkel zu begrenzen besteht darin, bei der Nachbearbeitung den Verbundwerkstoff zwischen den Gelenkplatten 5,6 nicht restlos wegzuschleifen. Das ergibt automatisch eine eingeschränkte Bewegungsfreiheit für die Gelenkplatten und somit einen begrenzten Beugewinkel. Wenn nach einer gewissen Zeit der Patient das Knie stärker biegen darf, braucht man lediglich den Verbundwerkstoff weiter abzuschleifen. Die zweite Möglichkeit besteht darin, in den dafür vorgesehenen Bohrungen 19 der oberen Gelenkplatten 6 (Fig. 3) eine Anschlagschraube 20 (Zylinderkopfschraube oder Madenschraube) einzusetzen und deren Gewinde auf der Innenseite der Knieorthese so weit herausstehen zu lassen, dass sie als Anschlag für den inneren Schenkel 14 der Viergelenkkette dient. Natürlich wird darauf geachtet, dass die Schraube gegen Herausdrehen gesichert ist und die Haut des Knies nicht verletzen kann. Es ist nun ersichtlich, dass die Streckbewegung begrenzt wird, wenn beim Strecken des Knies die obere Gelenkplatte 6 sich in Richtung der unteren Gelenkplatte 5 bewegt, was aber dank der Schraube 20 nur bis zu einem gewissen Punkt möglich ist. In Figur 4c wird gezeigt, wie beim Strecken des Knies in Richtung 23 die Oberkante 24 des inneren Schenkels 16 an der Anschlagschraube 20 ansteht und so den Streckwinkel 25 begrenzt, den wir auch zwischen den Flanken 18 beider Gelenkplatten wiederfinden. Zur weiteren Verdeutlichung zeigt Fig. 4b die Lage der Gelenkplatten 5,6 bei einem Beugewinkel von 0° (obere Gelenkplatte 6 ausgezogen) und bei einem beliebigen Beugewinkel (obere Gelenkplatte 6 strichpunktiert). Der Streckwinkel 25 und der Beugewinkel 22 kann individuell angepasst werden, da man lediglich die Lage der Bohrung 19 wählen und bestimmen muss, auf welche Seite das Gewinde hervorstehen soll. Dabei versteht es sich von selbst, dass eine der beiden Viergelenkketten 21 den Beugewinkel begrenzt, während dem die andere den Streckwinkel limitiert.

Dank der neuen Knieorthese in Verbundschichtbauweise mit integrierten überschlagenen Viergelenkketten nach Menschik können Patienten früher aus der Spitalpflege entlassen werden. Der Heilungsprozess wird merklich beschleunigt, was dem Patienten die schnelle Reintegration in den Arbeitsprozess ermöglicht. Ausserdem können dank der ausgezeichneten Stabilität, die dem Knie verliehen wird, teure Nachbehandlungen entfallen. Gelenkarthrosen werden selten auftreten, womit die Wahrscheinlichkeit der nachträglichen sehr teuren Implantation eines künstlichen Kniegelenks auf ein Minimum sinkt. Die Kosten für eine solche Knieorthese werden also mehr als wettgemacht.

Durch das geringe Gewicht und die eng anliegende Form kann die neue Knieorthese problemlos auch unter Jeans getragen werden. Diese Vorteile werden auch von Sportlern aus allen Disziplinen geschätzt. Zum Beispiel passt die eng anliegende Knieorthese ohne Schwierigkeiten in den Rennanzug eines Skifahrers. Auch Eishockeyspieler, Ruderer, Tennisspieler, Gewichtheber und Fussballer, um nur einige zu nennen, werden die neue Knieorthese bestimmt präventiv verwenden, da sie das Kniegelenk wirksam schützt, hundertprozentig passt und wegen dem geringen Gewicht komfortabel zu tragen ist. Die neue Knieorthese wird alle bestehenden Produkte dieser Art ersetzen, da sie dank der systematischen Anwendung modernster Werkstoffe einen echten Fortschritt in der Orthopädietechnik darstellt.

### HINWEISHUMMERNVERZEICHNIS

- 1: Gipsabdruck (Positiv)
- 2: Grundrichtplatte
- 3: Loch
- 4: Haltestift
- 5: Untere Gelenkplatte
- 6: Obere Gelenkplatte
- 7: Knieorthese
- 8: Positionierstift
- 9: Bohrung
- 10: PVA-Folie
- 11: Kohlefasergeflechtmatte (Karbonlage)
- 12: Glasfasertrikot
- 13: Kniegelenkkontur
- 14: Durchführungsschlitz
- 15: Klettverschluss
- 16: Innerer Schenkel
- 17: Aeusserer Schenkel
- 18: Anschlagflanke
- 19: Bohrung für Anschlagschraube
- 20: Anschlagschraube
- 21: Viergelenkkette
- 22: Beugewinkel
- 23: Streckrichtung
- 24: Oberkante des inneren Schenkels 16
- 25: Streckwinkel

## Patentansprüche

1. Knieorthese (7) zur Stabilisierung des Bandapparates im Knie mit einer Befestigungseinrichtung zur Befestigung oberhalb und unterhalb des Knies und einer Viergelenkkette (21), dadurch **gekennzeichnet,** daß die Befestigungseinrichtung Kunststoffschalen aufweist, die eine Karbon-Verbundschichtbauweise aufweisen, in welcher an je einer dem Lateralbereich des Kniegelenks entsprechenden inneren und äußeren Stelle, an welcher die Viergelenkkette (21) vorliegt, eine untere Gelenkplatte (5) und eine obere Gelenkplatte (6) der Viergelenkkette (21) in der Karbon-Verbundschicht eingebettet positioniert sind, wobei die Längserstreckung der Gelenkplatten (5,6) jeweils geringer als deren Quererstreckung ist.

2. Knieorthese nach Anspruch 1, dadurch gekennzeichnet, dass die untere Gelenkplatte (5) sichelförmig ausgebildet ist.

3. Knieorthese nach Anspruch 1 oder 2, dadurch gekennzeichnet, dass die obere Gelenkplatte (6) trapezoidförmig ausgebildet ist.

4. Knieorthese nach einem der Ansprüche 1 bis 3, dadurch gekennzeichnet, dass sie zur Einfassung des Beines, vorzugsweise drei Viertel des Beines, vorgesehen ist.

5. Knieorthese nach einem der Ansprüche 1 bis 4, dadurch gekennzeichnet, dass die Gelenkplatten (5, 6) Bohrungen (9) besitzen.

6. Knieorthese nach einem der Ansprüche 1 bis 5, dadurch gekennzeichnet, dass zwei Karbon-Verbundschichten zwischen zwei Abdeckschichten vorliegen.

7. Knieorthese nach einem der Ansprüche 1 bis 6, dadurch gekennzeichnet, dass die Gelenkplatten (5, 6) entsprechend einem Beugewinkel (22) des Knies vorliegen.

8. Knieorthese nach Anspruch 7, dadurch gekennzeichnet, dass der Beugewinkel (22) zwischen 0° und 60°, vorzugsweise 20°, 30°, 40° oder 45° beträgt.

9. Knieorthese nach einem der Ansprüche 7 oder 8, dadurch gekennzeichnet, dass der Beugewinkel (22) begrenzt ist.

10. Knieorthese nach Anspruch 9, dadurch gekennzeichnet, dass der Beugewinkel (22) durch eine Anschlagschraube (20) begrenzt ist.

11. Verfahren zur Herstellung eines orthopädischen Stützapparates zum Stützen beispielsweise von menschlichen Gliedmaßen, bei welchem ein positiver Gipsabdruck (1) des Beines hergestellt wird, auf dem anschließend eine Karbon-Verbundschicht (11) aufgebracht wird, die mit einem Laminat ausgegossen und dann ausgehärtet wird, dadurch **gekennzeichnet,** daß der orthopädische Stützapparat als Knieorthese (7) zur Stabilisierung des Bandapparates im Knie (13) mit einer Viergelenkkette (21) ausgebildet wird und im Lateralbereich des Kniegelenkes, in welchem die Viergelenkkette (21) vorgesehen ist, mindestens eine Grundrichtplatte (2) in den Gipsabdruck (1) eingesetzt wird, daß über die besagte Grundrichtplatte (2) die besagte Karbon-Verbundschicht (11) aufgebracht wird, daß an der besagten Grundrichtplatte (2) eine untere und eine obere Gelenkplatte (5, 6) der Viergelenkkette (21) positioniert werden und daß die besagte Grundrichtplatte (2) beim Ausschneiden der Kniegelenkkonturen und der Beseitigung des Gipsabdruckes entfernt wird.

12. Verfahren zur Herstellung eines orthopädischen Stützapparates zum Stützen beispielsweise von menschlichen Gliedmaßen, bei welchem ein positiver Gipsabdruck (1) des Beines hergestellt wird, auf dem anschließend eine Karbon-Verbundschicht (11) aufgebracht wird, die mit einem Laminat ausgegossen und dann ausgehärtet wird, dadurch **gekennzeichnet,** daß der orthopädische Stützapparat als Knieorthese (7) zur Stabilisierung des Bandapparates im Knie (13) mit einer Viergelenkkette (21) ausgebildet wird und im Lateralbereich des Kniegelenkes, in welchem die Viergelenkkette (21) vorgesehen ist, mindestens eine Grundrichtplatte (2) in den Gipsabdruck (1) eingesetzt wird, daß über die besagte Grundrichtplatte (2) die besagte Karbon-Verbundschicht (11) aufgebracht wird, daß an der besagten Grundrichtplatte (2) eine untere und eine obere Gelenkplatte (5, 6) der Viergelenkkette (21) positioniert werden, daß die besagte Grundrichtplatte (2) beim Ausschneiden der Kniegelenkkonturen (13) und der Beseitigung des Gipsabdruckes (1) entfernt wird und daß die starre Orthese zwischen den Gelenkplatten (5, 6) zerteilt wird, so daß eine obere und eine untere Hälfte anfallen und innere und äußere Schenkel zur Viergelenkkette (21) verbunden werden.

13. Verfahren nach Anspruch 11 oder 12, dadurch gekennzeichnet, dass zwei Grundrichtplatten eingesetzt werden.

14. Verfahren nach einem der Ansprüche 11 bis 13, dadurch gekennzeichnet, dass die Grundrichtplatte mit Haltestiften in zwei Löcher im positiven Gipsabdruck eingebracht wird.

15. Verfahren nach einem der Ansprüche 11 bis 14, dadurch gekennzeichnet, dass der Hohlraum zwischen dem Gipsabdruck und der Grundrichtplatte mit einem plastischen Werkstoff, z. B. Plastilin, ausgefüllt wird.

16. Verfahren nach einem der Ansprüche 11 bis 15, dadurch gekennzeichnet, dass eine erste Lage des Karbon-Verbundstoffes auf den Gipsabdruck aufgebracht wird, die untere und die obere Gelenkplatte eingesetzt wird, und eine zweite Karbon-Verbundschichtlage aufgebracht wird.

17. Verfahren nach Anspruch 16, dadurch gekennzeichnet, dass die untere und die obere Gelenkplatte auf Positionierstiften der Grundrichtplatte aufgesteckt werden, welche durch die erste Karbon-Verbundschicht hindurchragen.

18. Verfahren nach einem der Ansprüche 11 bis 17, dadurch gekennzeichnet, dass zunächst eine Folie, z. B. eine Kunststoff-Folie, auf den Gipsabdruck und über die Grundrichtplatte aufgebracht wird, danach die erste Lage der Karbon-Verbundschicht aufgebracht wird und anschliessend die Gelenkplatten auf der Grundrichtplatte befestigt werden und danach die zweite Lage der Karbon-Verbundschicht aufgebracht wird, auf welche eine Deckschicht, z. B. in Form einer Kunststoff-Folie, aufgebracht wird.

19. Verfahren nach einem der Ansprüche 16 bis 18, dadurch gekennzeichnet, dass die erste und die zweite Lage der Karbon-Verbundschicht eine Kohlefasergeflechtmatte ist.

20. Verfahren nach einem der Ansprüche 16 bis 19, dadurch gekennzeichnet, dass die untere und die obere Gelenkplatte auf eine Glasfaserschicht, z. B. eine Glasfasertrikotschicht, geklebt wird, welche vor dem Anordnen der Gelenkplatten auf die erste Lage der Karbon-Verbundschicht aufgebracht wird.

21. Verfahren nach einem der Ansprüche 18 bis 20, dadurch gekennzeichnet, dass das flüssige Laminat in die Karbon-Verbundschicht-Lagen zwischen der Kunststoff-Folie auf dem Gipsabdruck und der Deckschicht eingebracht wird.

22. Verfahren nach einem der Ansprüche 11 bis 21, dadurch gekennzeichnet, dass die mindestens eine Grundrichtplatte für einen Beugewinkel des Knies von 0° bis 60°, vorzugsweise 20°, 30°, 40° oder 45°, hergestellt ist.

## Claims

1. A knee orthesis appliance (7) for the stabilization of the ligaments in the knee with a fastening device for fastening above and below the knee and a four-bar mechanism (21), characterised in that the fastening device consists of a plastic shell, which is made of a carbon-fibre composite layer construction, in which, on both the inner and outer positions of the lateral area of the knee joint, which the four-bar mechanism (21) is in front of, a lower joint plate (5) and an upper joint plate (6) of the four-bar mechanism (21) have been embedded in position in the carbon-fibre composite layer, whereby the longitudinal extension of the joint plates (5,6) is less than their transverse extension.

2. A knee orthesis appliance in accordance with Claim 1, characterised in that the lower joint plate (6) is crescent-shaped.

3. A knee orthesis appliance in accordance with Claim 1 or 2, characterised in that the upper joint plate (6) is trapezoidal.

4. A knee orthesis appliance in accordance with Claims 1 to 3, characterised in that three-quarters of the leg is preferably involved in order to support the leg.

5. A knee orthesis appliance in accordance with claims 1 to 4, characterised in that the joint plates (5,6) have boreholes (9).

6. A knee orthesis appliance in accordance with Claims 1 to 5, characterised in that two carbon-fibre composite layers are located between the covering layers.

7. A knee orthesis appliance in accordance with Claims 1 to 6, characterised in that the joint plates (5,6) correspond to an angle of bend of the knee.

8. A knee orthesis appliance in accordance with claims 1 to 7, characterised in that the angle of bend is between 0° and 60°, but preferably 20°, 30°, 40°, or 45°.

9. A knee orthesis appliance in accordance with Claims 1 to 8, characterised in that the angle of bend is limited.

10. A knee orthesis appliance in accordance with Claims 1 to 9, characterised in that the angle of bend is limited by a stop screw (20).

11. A method for the manufacture of an orthopaedic support device for supporting, for example, human limbs, with which a positive plaster impression (1) of the leg is made on which finally a carbon-fibre composite layer (11) is applied, which is poured with a laminate and then hardened, characterised in that the orthopaedic support device is designed as a knee orthesis (7) for stabilizing the ligaments of the knee (13) with a four-bar mechanism (21) and in the lateral area of the knee joint, in which the four-bar mechanism (21) is provided, at least one basic shaping plate (2) is inserted into the plaster impression (1), that the mentioned carbon-fibre composite layer (11) is positioned over the mentioned basic shaping plate (2), that the lower and upper joint plates (5,6) of the four-bar mechanism (21) are positioned on the mentioned basic shaping plate (2) and the mentioned basic shaping plate (2) is removed when cutting out the knee joint contour and the removal of the plaster impression.

12. A method for the manufacture of an orthopaedic support device for supporting, for example, human limbs, with which a positive plaster impression (1) of the leg is made on which finally a carbon-fibre composite layer (11) is applied, which is poured with a laminate and then hardened, characterised in that the orthopaedic support device is designed as a knee orthesis (7) for stabilizing the ligaments of the knee (13) with a four-bar mechanism (21) and in the lateral area of the knee joint, in which the four-bar mechanism (21) is provided, at least one basic shaping plate (2) is inserted into the plaster impression (1), that the mentioned carbon-fibre composite layer (11) is positioned over the mentioned basic shaping plate (2), that the lower and upper joint plates (5,6) of the four-bar mechanism (21) are positioned on the mentioned basic shaping plate (2) and the mentioned basic shaping plate (2) is removed when cutting out the knee joint contour and the removal of the plaster impression and the rigid orthesis is divided between the joint plates (5,6) creating an upper and a lower half and the inner and outer legs are connected to the four-bar mechanism (21).

13. A process in accordance with Claims 11 to 12, characterised in that two basic shaping plates are used.

14. A process in accordance with one of the Claims 11 to 13, characterised in that the basic shaping plates are fixed in the positive plaster cast with retaining pins.

15. A process in accordance with one of the Claims 11 to 14, characterised in that the hollow space between the plaster cast and the basic shaping plate are filled with a plastic material, e.g. Plasticine.

16. A process in accordance with one of the Claims 11 to 15, characterised in that an initial layer of carbon-fibre composite material is applied to the plaster cast, the lower and the upper joint plate is fitted and a second carbon-fibre composite layer is applied.

17. A process in accordance with Claim 16, characterised in that the lower and the upper joint plates are located on positioning pins in the basic shaping plate, which extend through the first carbon-fibre composite layer.

18. A process in accordance with one of the Claims 11 to 17, characterised in that a foil, e.g. a plastic film, is applied to the plaster cast and over the basic shaping plate, after which the first carbon-fibre composite layer is applied and then the joint plates are attached to the basic shaping plate, whereafter the second carbon-fibre composite layer is applied, to which a covering layer in the form of a plastic film is applied.

19. A process in accordance with one of the Claims 16 to 18, characterised in that the first and second layers of the carbon-fibre composite layer are a carbon-fibre fibre net matting.

20. A process in accordance with one of the Claims 16 to 19, characterised in that the lower and upper joint plates are glued onto a layer of glass fibre, e.g. knitted glass fibre, which is applied to the first carbon-fibre composite layer prior to the joint plates being positioned.

21. A process in accordance with one of the Claims 18 to 20, characterised in that the fluid laminate is poured into the carbon-fibre composite layers between the plastic foil on the plaster cast and the covering layer.

22. A process in accordance with one of the Claims 11 to 21, characterised in that at least one basic shaping plate is made for a knee angle of bend of 0° to 60°, but preferably 20°, 30°, 40°, or 45°.

## Revendications

1. Orthèse de genou (7) destinée à stabiliser les ligaments du genou grâce à un dispositif de fixation placé au-dessus et au-dessous du genou ainsi qu'à une chaîne cinématique à 4 joints articulés (21), **caractérisée** par le fait que le dispositif de fixation se compose de coupelles synthétiques formées de couches composites en carbone dans lesquelles sont intégrées une plaque articulée inférieure (5) et une plaque articulée supérieure (6) de la chaîne cinématique à 4 joints articulés (21) placées dans chacune des zones latérales de l'articulation du genou aux points intérieur et extérieur où la chaîne cinématique à 4 joints articulés (21) est fixée; l'extension longitudinale des plaques articulées (5, 6) étant chaque fois plus étroite que leur extension transversale.

2. Orthèse de genou selon revendication 1, caractérisée par le fait que la plaque articulée inférieure (5) est en forme de croissant.

3. Orthèse de genou selon revendications 1 ou 2, caractérisée par le fait que la plaque articulée supérieure (6) est de forme trapézoïdale.

4. Orthèse de genou selon une des revendications 1 à 3, caractérisée par le fait qu'elle est prévue pour entourer la jambe, de préférence aux trois quarts.

5. Orthèse de genou selon une des revendications 1 à 4, caractérisée par le fait que les plaques articulées (5, 6) sont pourvues d'alésages (9).

6. Orthèse de genou selon une des revendications 1 à 5, caractérisée par le fait que deux couches composites en carbone sont placées entre deux couches de recouvrement.

7. Orthèse de genou selon une des revendications 1 à 6, caractérisée par le fait que les plaques articulées (5, 6) forment un angle de pliage correspondant (22) du genou.

8. Orthèse de genou selon revendication 7, caractérisée par le fait que l'angle de pliage (22) se situe entre 0° et 60°, de préférence à 20°, 30°, 40° ou 45°.

9. Orthèse de genou selon une des revendications 7 ou 8, caractérisée par le fait que l'angle de pliage (22) est limité.

10. Orthèse de genou selon revendication 9, caractérisée par le fait que l'angle de pliage (22) est limité par une vis de butée (20).

11. Procédé de fabrication d'un appareil de soutien orthopédique, destiné par exemple à soutenir des membres humains, pour lequel un moulage en plâtre positif (1) de jambe est fabriqué, sur lequel est placé ensuite une couche composite en carbone (11), coulée avec un laminé et ensuite durcie, **caractérisé** par le fait que l'appareil de soutien orthopédique destiné à stabiliser les ligaments du genou (13) est conçu comme une orthèse de genou (7) comprenant une chaîne cinématique à 4 joints articulés (21) et qui, dans la zone latérale de l'articulation du genou dans laquelle la chaîne cinématique à 4 joints articulés (21) est prévue, comprend au moins une plaque de redressement de base (2) intégrée dans le moulage en plâtre (1), par le fait aussi que ladite couche composite en carbone (11) est placée au-dessus de ladite plaque de redressement de base (2), que des plaques articulées inférieure et supérieure (5, 6) de la chaîne cinématique à 4 joints articulés (21) sont positionnées sur ladite plaque de redressement de base (2), et que ladite plaque de redressement de base (2) est enlevée lorsque l'on extirpe les contours de l'articulation du genou et que l'on ôte le moulage en plâtre.

12. Procédé de fabrication d'un appareil de soutien orthopédique, destiné par exemple à soutenir des membres humains, pour lequel un moulage en plâtre positif (1) de jambe est fabriqué, sur lequel est placé ensuite une couche composite en carbone (11), coulée avec un laminé et ensuite durcie, **caractérisé** par le fait que l'appareil de soutien orthopédique destiné à stabiliser les ligaments du genou (13) est conçu comme une orthèse de genou (7) comprenant une chaîne cinématique à 4 joints articulés (21) et qui, dans la zone latérale de l'articulation du genou dans laquelle la chaîne cinématique à 4 joints articulés (21) est prévue, comprend au moins une plaque de redressement de base (2) intégrée dans le moulage en plâtre (1), par le fait aussi que ladite couche composite en carbone (11) est placée au-dessus de ladite plaque de redressement de base (2), que des plaques articulées inférieure et supérieure (5, 6) de la chaîne cinématique à 4 joints articulés (21) sont positionnées sur ladite plaque de redressement de base (2), et que ladite plaque de redressement de base (2) est enlevée lorsque l'on extirpe les contours de l'articulation du genou (13) et que l'on ôte le moulage en plâtre (1), et que l'orthèse rigide entre les plaques articulées (5, 6) est divisée de façon à obtenir une moitié supérieure et inférieure, et que les côtés intérieurs et extérieurs soient reliés à la chaîne cinématique à 4 joints articulés (21).

13. Procédé selon revendications 11 ou 12, caractérisé par le fait que deux plaques de redressement de base sont mises en place.

14. Procédé selon une des revendications 11 à 13, caractérisé par le fait que la plaque de redressement de base est mise en place à l'aide de goupilles de maintien insérées dans deux trous du moulage en plâtre positif.

15. Procédér selon une des revendications 11 à 14, caractérisé par le fait que l'espace vide situé entre le moulage en plâtre et la plaque de redressement de base est rempli d'une matière plastique, par exemple plastiline.

16. Procédé selon une des revendications 11 à 15, caractérisé par le fait qu'une première couche de la matière composite en carbone est appliquée sur le moulage en plâtre, que les plaques articulées inférieure et supérieure sont intégrées, et qu'une deuxième couche composite en carbone est ensuite appliquée.

17. Procédé selon revendication 16, caractérisé par le fait que les plaques articulées inférieure et supérieure sont fixées sur les goupilles de positionnement de la plaque de redressement de base, lesquelles traversent la première couche composite en carbone.

18. Procédé selon une des revendications 11 à 17, caractérisé par le fait qu'une feuille, par exemple une feuille synthétique, est tout d'abord appliquée sur le moulage en plâtre et au-dessus de la plaque de redressement de base, qu'ensuite la première couche composée en carbone est appliquée, que les plaques articulées sont alors fixées sur la plaque de redressement de base, que l'on applique ensuite la deuxième couche du composé en carbone sur laquelle l'on applique à son tour une couche de recouvrement, par exemple sous forme d'une feuille synthétique.

19. Procédé selon une des revendications 16 à 18, caractérisé par le fait que la première et la deuxième couche du composite en carbone sont constituées par un tressage en fibre de carbone.

20. Procédé selon une des revendications 16 à 19, caractérisé par le fait que les plaques articulées inférieure et supérieure sont collées sur une couche en fibre de verre, par exemple un tissage en libre de verre, laquelle est appliquée sur la première couche du composé en carbone avant la mise en place des plaques articulées.

21. Procédé selon une des revendications 18 à 20, caractérisé par le fait que le laminé liquide est intégré dans les couches du composé en carbone entre la feuille synthétique sur le moulage en plâtre et la couche de recouvrement.

22. Procédé selon une des revendications 11 à 21, caractérisé par le fait qu'au moins une plaque de redressement de base est fabriquée pour un angle de pliage du genou de 0° à 60°, de préférence 20°, 30°, 40° ou 45°.
